# EUROPEAN PATENT APPLICATION

(11) **EP 3 205 302 A1**
(43) Date of publication of application: **16.08.2017**
(21) Application number: 15848352.9
(22) Date of filing: 08.10.2015
(51) Int. Cl.: A61B 18/12

(54) **ABLATION CATHETER DEVICE**

(30) Priority: 11.10.2014 CN 201410535076
(71) Applicant: Lifetech Scientific (Shenzhen) Co., Ltd., Shenzhen, Guangdong 518057 (CN)
(72) Inventor: ZHUANG, Shaochun, Shenzhen Guangdong 518057 (CN); CHEN, Wenjun, Shenzhen Guangdong 518057 (CN); LIU, Peng, Shenzhen Guangdong 518057 (CN); XIA, Shengping, Shenzhen Guangdong 518057 (CN)
(74) Representative: Prinz & Partner mbB
(86) International application number: PCT/CN2015/091461
(87) International publication number: WO 2016/055000

(57) **Abstract**

An ablation catheter device (100) comprises a hollow catheter main body (102), an ablation mechanism (103) and a control mechanism (105). The ablation mechanism (103) comprises a support assembly (110) capable of being expanded and compressed radially, an end (120) and a plurality of modulation units. The support assembly (110) is provided between the distal end of the catheter main body (102) and the end (120). The modulation units are provided on the support assembly (110), and an axial through hole (122) is formed in the end (120). The control mechanism (105) comprises a drawing wire (104) and a limit unit (118) fixed on the drawing wire (104). The drawing wire (104) axially penetrates the catheter main body (102) and the through hole (122). The end (120) is provided between the support assembly (110) and the limit unit (118), and the outer diameter of the limit unit (118) is larger than the pore diameter of the through hole (122). The ablation catheter device (100) is suitable for a transfemoral coronary puncture intervention path, or is preferably suitable for a transradial coronary puncture intervention path. For some bent and complex artery blood vessels, the ablation catheter device (100) can reduce the adjustment and moving location difficulty in the blood vessels, avoid damages to the blood vessels as much as possible and improve the ablation effect.

## Description

### Technical Field

The invention relates to the field of medical devices and instruments, and particularly to an ablation catheter device.

### Background

Hypertension is a common chronic disease. In the formation mechanism of hypertension, renin-angiotensin-aldosterone system (RAAS), as an important blood pressure regulating system, plays a significant role, and it maintains balance of water, electrolytes and blood pressure of a human body by regulating the heart, the blood vessels and the kidneys. It has been proved through researches that RAAS causes hypertension in the following three ways: (1) sodium retention due to RAAS activation; (2) activity of sympathetic nervous system can be increased through RAAS activation; and (3) blood vessels can be directly contracted through RAAS activation. Wherein, renal artery sympathetic nerve plays a decisive role in inducing and maintaining systematic hypertension, and an overactivity of the renal artery sympathetic nerve results in maintenance of hypertension.

Transcatheter renal artery sympathetic nerve ablation (RDN) is an ablation strategy for the renal artery sympathetic nerve. A typical way that the ablation works is to transport the modulation units, such as electrodes, ultrasonic transducers and medicine and the like to the renal artery blood vessels of the body of a patient through a catheter, and applying energies, such as electric energy, sound energy, heat energy and the like to the renal artery blood vessels through the modulation units to achieve the purpose of ablating the renal sympathetic nerve in the renal artery blood vessel walls so as to reduce the blood pressure of the patient.

In the prior art, the radiofrequency ablation catheter is only provided with an individual modulation unit generally, for example, an electrode is mounted at a distal end of the main body of the catheter. The distal end of the main body of the catheter is bent by virtue of handle control, so that the electrode clings to the inner wall of the blood vessel and forms a circular loop with a neutral electrode plate on the body surface, so as to ablate the renal sympathetic nerve. That way is simple, and the guiding catheter (sheath) needed to be cooperated in use is sized smaller. But it would be unstable for the electrode to be clung to the inner wall of the blood vessel. With pulsation of the blood vessel, the electrode would be separated from the wall of the blood vessel. Furthermore, ablation at multiple places of the inner wall of the blood vessel is needed for blockage of the renal sympathetic nerve. The position of the electrode needs to be adjusted many times in use, which prolongs the operation time of the patient, and the case that a same target spot is repeatedly ablated is also likely to happen to lead to severe damages of the wall of the blood vessel.

Therefore, a plurality of modulation units are provided for solving the problems at present. In the ablation process, the components provided with the modulation units are expanded by virtue of handle control, so that this manner ensures that each electrode (modulation unit) can be stably clung to the renal artery blood vessel wall, the time for adjusting the position of the electrode is reduced, and the operation time for the patient is shortened as well. However, the expandable structure provided with the electrodes makes the size of the distal end of the main body of the catheter be obviously increased relative to that of an individual electrode structure. The ablation catheter needs to be cooperated with the guiding catheter or the transporting sheath which is sized larger in use, for example, the sheath which is larger than6F (1 mm=3F). The electrodes cannot be transported in the guiding catheter or the transporting sheath which is sized as 6F or smaller than 6F; on the other hand, the structure increases the rigidity of the distal end of the main body of the catheter, so that the operating difficulty that the distal end of the main body of the catheter entering or exiting the guiding catheter or the transporting sheath and the operating difficulty in the transporting process is likely to happen.

It is well-known that the transporting sheath which is sized larger than 6F is hardly used through radial artery intervention but is often used through femoral artery intervention. Compared with femoral artery puncture, radial artery puncture causes slighter vascular puncture point wounds and puncture point complications; on the other hand, radial artery puncture is shorter in postoperative nursing time. Meanwhile, as far as patients with bilateral coxofemoral deformation, patients who are too obese to be given femoral artery puncture and are hardly stopped in bleeding, patients who suffer cardiac insufficiency and cannot lie on the back for a long time as a result of pathological changes of lumbar vertebra and patients with medical history of lower limb deep vein thrombus or pulmonary embolism are concerned, femoral artery puncture is not suitable while radial artery puncture shall be taken into consideration for those patients.

In addition, as can be known from the anatomical structures of the blood vessels, the renal artery blood vessels are different in shapes: some blood vessels are relatively straight while some are relatively bent and complex. For the relatively straight renal artery blood vessels, in the operation process, the distal end of the main body of a catheter can be smoothly adjusted and moved in the blood vessels; while for the relatively bent and complex renal artery blood vessels, in the operation process, ablation is hardly accomplished or the inner walls of the blood vessels of the patient are damaged due to the fact that the distal end of the main body of the catheter is hard to be adjusted, and movably positioned in the renal artery blood vessels because of both the larger size and greater rigidity of the distal end of the main body of the catheter.

### Summary of the Invention

Thus, it is necessary to provide an ablation catheter device; not only the transfemoral artery puncture intervention path, but also the transradial artery puncture intervention path which is more advantageous and can be selected when the ablation catheter device is used for operation. Moreover, for some bent and complex artery blood vessels, for example, the renal artery blood vessels, the ablation catheter device can reduce the adjustment and moving location difficulty in the blood vessels, avoid damages to the blood vessels as much as possible and improve the ablation effect.

An ablation catheter device, comprising
A hollow catheter main body;
An ablation mechanism including a support assembly capable of being expanded and compressed radially, an end and a plurality of modulation units, wherein the support assembly is provided between the distal end of the main body of the catheter and the end, the plurality of the modulation units are provided on the support assembly, and the end has an axial through hole; and

A control mechanism which comprises a drawing wire and a limit unit fixed on the drawing wire, wherein the drawing wire penetrates the main body of the catheter and the through hole coaxially and movably, the end is provided between the support assembly and the limit unit, and the outer diameter of the limit unit is larger than the pore diameter of the through hole.

In one of the embodiments, the support assembly comprises a plurality of elastic support rods isolated from each other, the proximal ends of the support rods are connected to the distal ends of the main body of the catheter, and the distal ends of the support rods are all connected to the end.

In one of the embodiments, the support rod comprises a top which is approximately parallel to the main body of the catheter, two inclined parts which incline relative to the main body of the catheter and two bending parts; the modulation units are provided on the top; the two inclined parts are provided on two sides of the top respectively, and are connected to the main body of the catheter and the end respectively; the two bending parts are connected to the top and the two inclined parts, respectively; and the rigidity of the bending parts is smaller than that of the top and the inclined parts.

In one of the embodiments, the cross section of the top which is perpendicular to the length direction of the main body of the catheter is arc-shaped.

In one of the embodiments, tops of the plurality of support rods cooperate with each other to form an approximately circular ring on the cross section which is perpendicular to the length direction of the main body of the catheter; and the drawing wire movably penetrates the circular ring.

In one of the embodiments, on the cross section which is perpendicular to the length direction of the main body of the catheter, the widths of the tops are maximum and the widths of the inclined parts are minimum, and the widths of the bending parts gradually narrow from the tops to the inclined parts.

In one of the embodiments, the tops are coated with insulating layers, and the modulation units are provided on the outer surface of the insulating layers.

In one of the embodiments, the end comprises an axially provided groove which receives the distal ends of the support rods.

In one of the embodiments, the drawing wire is a metal filament, and comprises a flexible distal end; the flexible distal end comprises a winding spring or a polymer sheath.

In one of the embodiments, the ablation catheter device further comprises a handle provided at the proximal end of the main body of the catheter, and the drawing wire is controlled by the handle to move axially.

Compared with the ablation catheter device in the prior art, the ablation catheter device provided by the invention can realize radial dilation and radial compression of the support assembly by cooperating the drawing wire and limit unit with the end. The ablation catheter device is simple in structure and easy to operate without the need of other complex mechanical dilation and compression structures; The drawing wire, which is in a elongate soft structure, takes a small space, and substantially has no influence on the structural size of the support assembly, so that a material which is properly sized is selected and is mechanically processed a support assembly which can smoothly get in and out of and smoothly transported in the guiding catheter sized as 6F or smaller than 6F, so that it can be realized that the ablation catheter device can be intervened into therapy not only through transfemoral coronary puncture but also through transradial coronary puncture. Furthermore, the drawing wire in the control mechanism is flexible and the length can be selectively provided as needed, so that the drawing wire can be directly used as the guiding wire to establish the transporting path in the renal artery blood vessel, and is particularly suitable for moving in some bent and complex blood vessels, and can reduce the adjustment and moving location difficulty in the blood vessels, avoid damages to the blood vessels as much as possible and improve the ablation effect.

### Brief Description of the Drawings

Fig. 1A is a structural schematic diagram of the ablation catheter device;
Fig. 1B is a structural schematic diagram of the ablation catheter device in another state;
Fig. 2 is a structural schematic diagram of the support assembly positioned at the distal end of the main body of the catheter in the ablation catheter device;
Fig. 3A is a front view of structural schematic diagram of the support rod in the ablation catheter device;
Fig. 3B is a top view of structural schematic diagram of the support rod in the ablation catheter device;
Fig. 3C is a sectional schematic diagram of the support rod in the ablation catheter device;
Fig. 3D is a sectional schematic diagram of the support rod combined with the electrode and the insulating layer in the ablation catheter device;
Fig. 4A is a side view of schematic diagram of the end positioned at the distal end of the support assembly in the ablation catheter device;
Fig. 4B is a section view of schematic diagram of the end positioned at the distal end of the support assembly in the ablation catheter device;
Fig. 5 is a structural schematic diagram of an assembling structure of the end and the support assembly in the ablation catheter device;
Fig. 6 is a schematic diagram of the drawing wire in the ablation catheter device;
Fig. 7 is a sectional schematic diagram of the compression state of the support assembly in the ablation catheter device;
Figs. 8A, 8B and 8C are schematic diagrams of conditions of the ablation catheter device used in the bent and complex blood vessels;
Figs. 9A, 9B and 9C are schematic diagrams of conditions of the drawing wire used in the contralateral renal artery blood vessel in the ablation catheter device.

### Detailed Description of the Invention

To make the above objects, features and advantages of the present disclosure more obvious and easy to understand, the embodiments of the present disclosure will be further described in detail below in conjunction with the accompanying drawings and particular embodiments. In the following description, a number of details are set forth to provide a thorough understanding of the present disclosure. But the invention can be carried out by other means which are different from the description, and those skilled in the art can make similar modifications without departing from the inventive connotation, so that the invention is thus not limited by the following disclosed specific embodiments.

It will be understood that when an element is referred to as being "fixed on" another element, it can be directly on the other element or intervening elements may also be present; it will also be understood that when an element is referred to as being "connected " to another element, it can be directly connected to the other element or intervening elements may be present. In the intervention field, the end which is relatively proximate to the operator is called the proximal end while the end which is relatively distant from the operator is called the distal end.

Unless otherwise defined, all technical and scientific terms used herein have the same meaning as commonly understood by one of those skilled in the art to which this invention belongs. The terminology used in the description of the invention herein is for the purpose of describing particular embodiments only and is not intended to be limiting of the invention. As used herein, the term "and/or" comprises any and all combinations of one or more of the associated listed items.

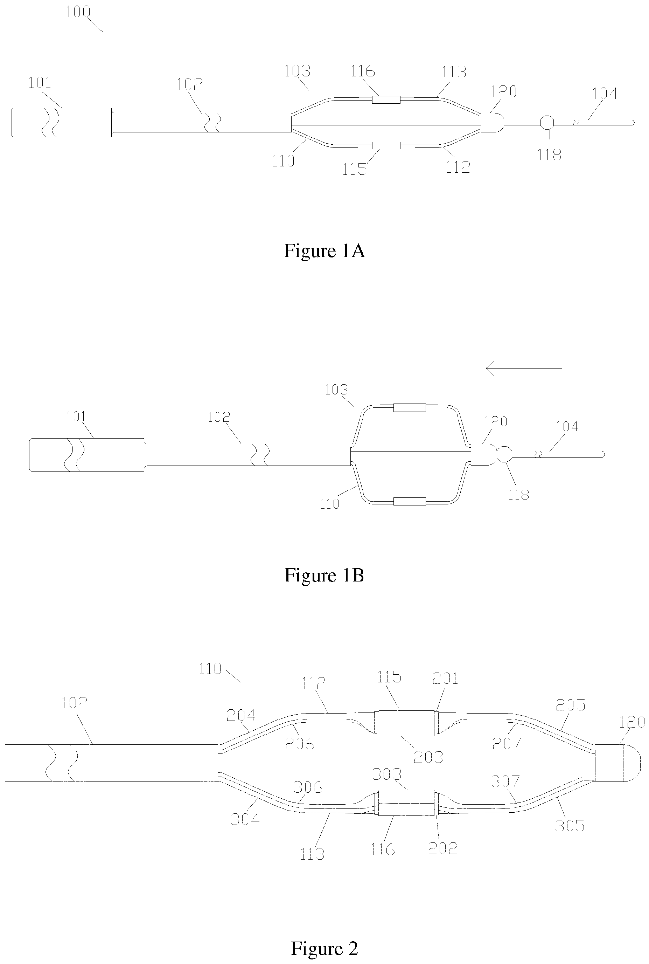

The Fig. 1A shows the structural schematic diagram of the ablation catheter device 100 disclosed by the invention. The ablation catheter device (hereinafter referred to as catheter 100) comprises the main body of the catheter 102, the ablation mechanism 103 and the control mechanism 105.

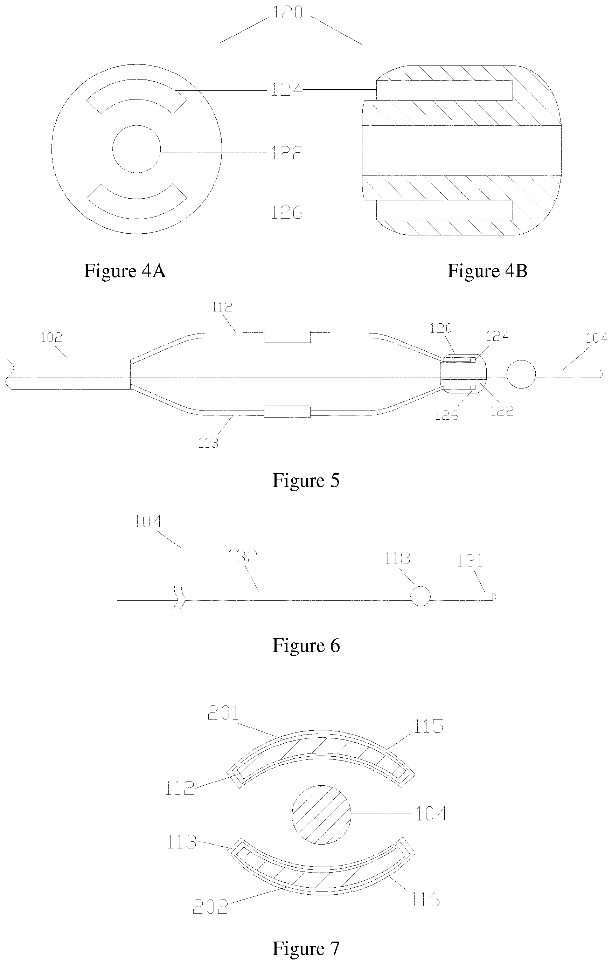

The main body of the catheter 102 is in a slender tube shape, axially extends from the proximal end to the distal end, and is hollow inside to form an axial inner cavity. The ablation mechanism 103 comprises a support assembly 110 capable of being expanded and compressed radially, an end 120 and a plurality of modulation units. The support assembly 110 comprises a plurality of elastic support rods 112 and 113, and the proximal ends of the elastic support rods 112 and 113 are connected to the main body of the catheter 102 and the distal ends of the elastic support rods 112 and 113 are connected to the end 120. The end 120 has an axially provided through hole 122 (Figs. 4A and 4B). The modulation units (taking electrodes 115 and 116 shown in the embodiments as an example) are provided on the support rods 112 and 113, respectively. The modulation units are connected to an energy generator (not shown) through wires (not shown) in the main body of the catheter 102, a connector (not shown) positioned on the handle 101 at the proximal end of the main body of the catheter 102 and a connecting cable (not shown). When the energy generator emits radio frequency energy, the electrodes 115 and 116 in the blood vessel and a body surface electrode (not shown, the body surface electrode is a neutral electrode plate which is generally placed on the surface of a patient, for example, back or thigh) will form a circle loop so as to form an electric field between the two electrodes to be acted on the tissue between the two electrodes and generate a thermal effect so as to ablate the tissue.

The control mechanism 105 comprises the drawing wire 104 and the limit unit 118 fixed on the drawing wire 104. The drawing wire 104 penetrates through the inner cavity of the main body of the catheter 102 and the through hole 122 of the end 120 axially and movably, the limit unit 118 is provided at the distal end relative to the end 120, and the outer diameter of the limit unit 118 is slightly larger than the pore diameter of the through hole 122 of the end 120. The handle 101 has members (not shown) such as a driving mechanism and a control mechanism which control the drawing wire 104 and the main body of the catheter 102 to move axially.

During use, the surgeon establishes a transporting path through a vascular puncture point, the support assembly 110 of the catheter 100 is pushed to the target blood vessel of the patient through the transporting path, and the drawing wire 104 is operated by the handle 101 to move toward the proximal end. Shown by the arrowhead in the Fig. 1B, the limit unit 118 blocks the distal end of the end 120, so that the drawing wire 104 cannot continuously move relative to the distal end of the support assembly 110. The drawing wire 104 is continuously operated to move toward the proximal end. As the limit unit 118 props against the distal end of the end 120, the pull is transferred to the support rods 112 and 113, so that the support rods are elastically bent, and therefore, the support assembly 110 forms an expanded structure, as shown in Fig. 1B. The electrodes 115 and 116 on the support rods 112 and 113 will cling to the wall of the blood vessel and transport energies to the blood vessel wall tissue. Particularly, when the target blood vessel is bent and complex in shape, it is hard for the surgeon to directly push the support assembly 110 of the catheter 100 to the target blood vessel after establishing the transporting path. At the moment, the surgeon can operate the control mechanism 105 through the handle 101 to make the drawing wire 104 move toward the distal end. After the drawing wire 104 firstly enters the target blood vessel and continuously moves forward for a section of distance, the surgeon then pushes the support assembly 110 to the target blood vessel along the drawing wire 104 and accomplishes ablation through a follow-up operation.

Fig. 2 shows a structure of more details of the ablation mechanism 103 at the distal end of the catheter 100. The ablation mechanism 103 comprises the supporting rods 112 and 113, the electrodes 115 and 116, the end 120, and the insulating layers 201 and 202 coated on the supporting rods 112 and 113.

The support rods 112 and 113 are totally the same in shapes and structures, are in axially symmetrical distribution, and are approximately arc-shaped. The distal ends of the support rods 112 and 113 are connected in the end 120 (the end is shown in the Figs. 4A and 4B and specific description below), respectively, while the proximal ends of the support rods are connected to the main body of the catheter 102. Each support rod of the support assembly 110 comprises a top which is approximately parallel to the main body of the catheter, two inclined parts which incline relative to the main body of the catheter and two bending parts; the two inclined parts are provided on two sides of the top respectively, and are connected to the main body of the catheter and the end, respectively; the two bending parts are connected to the top and the two inclined parts, respectively; and the rigidity of the bending parts is smaller than that of the top and the inclined parts.

For example, the support rod 112 comprises a top 203, an inclined part 204, an inclined part 205, a bending part 206 and a bending part 207. The support rod 113 further comprises a top 303, a inclined part 304, a inclined part 305, a bending part 306 and a bending part 307 as well. The electrodes 115 and 116 are provided on the surface of two tops of the support assembly 110, and the tops between the electrodes and the support rods have the insulating layers 201 and 202, so that in the ablation process, the electrodes and the support rods are electrically insulative, the insulating layers are made from PET, Pebax, PTFE, Parylene or a silica gel and the like, and the electrodes are made from gold, platinum, iridium and the like.

The support rods 112 and 113 are elastic and can be bent by external forces. For example, the support rods are made from a nickel and titanium material. The integral shape of the support assembly 110, by virtue of self-elasticity and the action of the handle 101, changes along with shape change of the support rods 112 and 113 so as to achieve an effect of radial expansion or radial compression. The shape of the support assembly 110 can be adjusted according to the size of the target blood vessel. When the size of the target blood vessel is moderate or relatively small, the support assembly 110 can make the electrodes cling to the wall of the blood vessel without the need of expanding to the maximum expanding size. When the size of the target blood vessel is relatively great, the support assembly 110 will expand to the maximum radial size at the moment, so that the electrodes can stably cling to the wall of the blood vessel. Therefore, the target blood vessels in various sizes can be suitable by controlling the radial expansion extent of the support assembly 110 so as to achieve the optimum ablation effect.

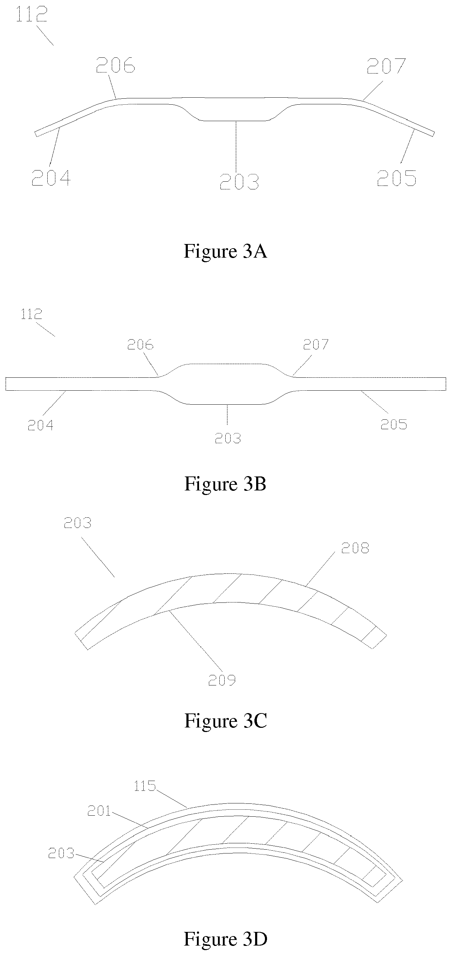

The elastic properties of the support assembly 110 are from members thereof, i.e. the support rods 112 and 113; Fig. 3A and Fig. 3B show more details about the structure of the support rod 112; and since the support rods 112 and 113 are identical in structure, the support rods 113 and 112 have the same features.

Generally, the support rods are formed by machining and molding a pipe fitting material to form a structure meeting the requirements on the properties of the support assembly, for example, the above pipe fitting material is a hyperelastic nickel-titanium material. As shown in Fig. 3A and Fig. 3B, Fig. 3A is a front view, and Fig. 3B is a top view; each support rod 112 comprises a top 203, an inclined part 204, an inclined part 205, a bending part 206 and a bending part 207; the top 203 of the support rod 112 is relatively wide relative to the inclined part 204, the inclined part 205, the bending part 206 and the bending part 207 and is the widest part of the support rod 112; the top 203 has a given length, so that the electrodes completely cover the surface of the top 203; and the width of the bending parts 206, 207 of the support rod 112 is gradually narrowed from the top 203 to the inclined parts 204, 205. It should be understood that the called "width" refers to an extension size of each part of the support rod 112 on a cross section, which is perpendicular to the length direction (i.e. the axial direction), of the main body of the catheter 102.

The rigidity of the bending parts 206, 207 of the support rod 112 is smaller than that of the top 203 and the inclined parts 204, 205 of the support rod 112, for example, the width and the thickness of the bending parts 206, 207 are changed in a way such as cutting, grinding, polishing and the like, so that the rigidity of the bending parts 206, 207 may be smaller than the rigidity of the top 203 and the inclined parts 204, 205. Therefore, by means of the elasticity of the support rod 112 and the function of a control member of the handle 101, the bending parts 206, 207 are bent, as shown in Fig. 3B. Fig. 3C shows a cross section structure, which is perpendicular to the length direction of the main body of the catheter 102, of the top 203 of the support rod 112; and the cross section of the top 203 of the support rod 112 is arc-shaped, and comprises an outer arc 208 and an inner arc 209. Since the support rod 112 is formed by machining the pipe fitting, the cross section of each position of the support rod 112 may be considered to be arc-shaped. When the insulation layer 201 and the electrode 115 are provided on the surface of the top 203, since the insulation layer 201 and the electrode 115 are very thin in thickness, the top 203 (comprising the insulation layer and the electrode) is still of an arc structure, as shown in Fig. 3D.

Fig. 4A and Fig. 4B show the structure of the end 120 disposed at the distal end of the support assembly 110. Fig. 4A is a front view of the end 120 seen from the proximal end, and Fig. 4B is an axial section view of the end 120. As shown in the figures, the end 120 is a cylinder with two chamfered ends, and is provided with a through hole 122, a groove 124 and a groove 126, wherein the through hole 122 penetrates through the whole end 120 in the axial direction; the inner diameter of the through hole 122 is slightly larger than the outer diameter of the drawing wire 104 but smaller than the outer diameter of the limit unit 118 at the distal end of the drawing wire 104, so that the main body of the drawing wire 104 penetrates the support assembly 110, the main body of the catheter 102 and the handle 101 via the through hole 122 of the end 120, while the limit unit 118 cannot pass through the through hole 122 of the end 120. The groove 124 and the groove 126 are symmetrically distributed in the circumferential direction of the periphery of the through hole 122 of the end 120; and openings of the grooves 124 and 126 and the cross sections of the inclined parts 204, 205 of the support rod 112 are the same in shape and are respectively arc-shaped; the grooves 124 and 126 have a given width and depth, for example, the grooves are sized larger than that of the cross section of the inclined parts of the support rods, so that the support rods 112 and 113 can be respectively inserted into the grooves 124 and 126 and are fixed in an adhesive way, a welding way and the like, and the support assembly 110 and the end 120 are fixedly connected with each other, as shown in Fig. 5.

In the present embodiment, the distal ends of the support rods 112 and 113 are separated by cutting the pipe fittings; and in another embodiment not shown, the proximal ends or the distal ends of the support rods 112 and 113 may also be closed and mutually connected together.

Fig. 6 shows the structure of the drawing wire 104; and the drawing wire is in an elongated shape and is made of a metal such as a nickel-titanium wire and a stainless steel wire. The drawing wire 104 comprises a main body 132 and a flexible distal end 131, wherein the flexible distal end 131 comprises an intertwined spring or a polymer jacket; and the polymer jacket may be a PTFE coating, so that when a doctor operates the handle 101 to push the drawing wire 104 to move towards the distal end, the drawing wire 104 can enter a target blood vessel, and particularly can easily move continuously in some curved and complicated target blood vessels.

As shown in Fig. 5, the outer diameter of the main body 132 of the drawing wire 104 is smaller than the inner diameter of the through hole 122 of the end 120, so that the main body 132 can pass through the end 120 and the main body of the catheter 102 to reach the handle 101, and can move and rotate in an inner cavity of the main body of the catheter 102. The outer diameter of the limit unit 118 fixedly provided on the drawing wire 104 is larger than the inner diameter of the through hole 122 of the end 120, so that when the surgeon operates the handle 101, the drawing wire 104 moves toward the proximal end until the limit unit 118 props against the end 120 and cannot continuously move relative to the end 120, and then the drawing wire 104 continuously move towards the proximal end; and since the force for operating the drawing wire 104 to move toward the proximal end and the blockage when the limit unit 118 encounters the end 120 would enable the bending parts of the support assembly 110 to be bent and deformed and would enable the support assembly 110 to be expanded in the radial direction, so that the electrodes cling to the wall. The limit unit 118 is made of metal or plastics and is in a circular shape, an elliptical shape or any shape capable of meeting the above requirements, and the like.

When the support assembly 110 of the catheter 100 is transported in a guiding catheter, the support assembly 110 is compressed into an elongated tubular shape, as shown in Fig. 7. The support rods 112 and 113 are preferably selected from two symmetrically distributed parts of a same pipe fitting and are also symmetrically distributed in the support assembly 110; and when the top of the support rod is provided with the insulation layer and the electrode, since the insulation layer and the electrode are very thin in thickness, the top of the support rod is still of an arc structure, as shown in Fig. 3D. When the support assembly is compressed into the elongated tubular shape, two tops, which are respectively provided with the electrode and the insulation layer, of the support rod 112 and the support rod 113 form an approximately circular ring on the cross section, such as a partially-missing circular ring; and the outer diameter of the circular ring is slightly larger than the outer diameter of the pipe fitting machined into the support rod, and the inner diameter is slightly smaller than the inner diameter of the above pipe fitting.

For example, the outer diameter of a pipe piece for cutting is 1.2 mm and the inner diameter is 0.8 mm, while the thickness by which the electrodes are closely clung to the insulating layers is 0.1 mm, the outer diameter of a tube structure formed after the compression of the support assembly 110 is 1.4 mm and the inner diameter is 0.6 mm, i.e. the inner diameter of a partially-missing circular ring formed by the cross section of the two tops is 0.6 mm, while the outer diameter of the selected drawing wire 104 is 0.3 mm, the outer diameter thereof is smaller than the inner diameter of the above mentioned circular ring, and the drawing wire 104 with such a size all along is freely moving within the support assembly 110 under compression. Thus, the drawing wire 104 does not increase the overall size of the support assembly 110 under compression, i.e. the outer diameter of the support assembly 110 after compression remains to be 1.4 mm, which is smaller than the inner diameter (1.78 mm) of the 6F (2 mm) guiding catheter. Therefore, the ablation catheter device sized as above of the present invention can smoothly get in and out of and transported in the guiding catheter of 6F. The size of the ablation catheter device of the present disclosure comprises but is not limited to the above mentioned sizes. Thus, pipe fittings which are properly sized are selected and are mechanically processed into a support rod which is further processed into a support assembly 110 after being disposed with the electrodes and the insulating layers, a support assembly 110 can smoothly get in and out of and transported in the guiding catheter sized as 6F or smaller than 6F, so that it can be realized that the catheter 100 can be intervened into therapy not only through transfemoral coronary puncture but also through transradial coronary puncture.

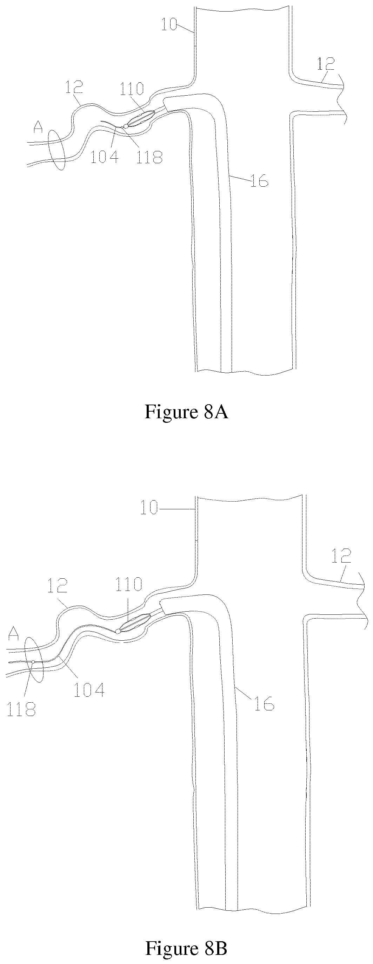

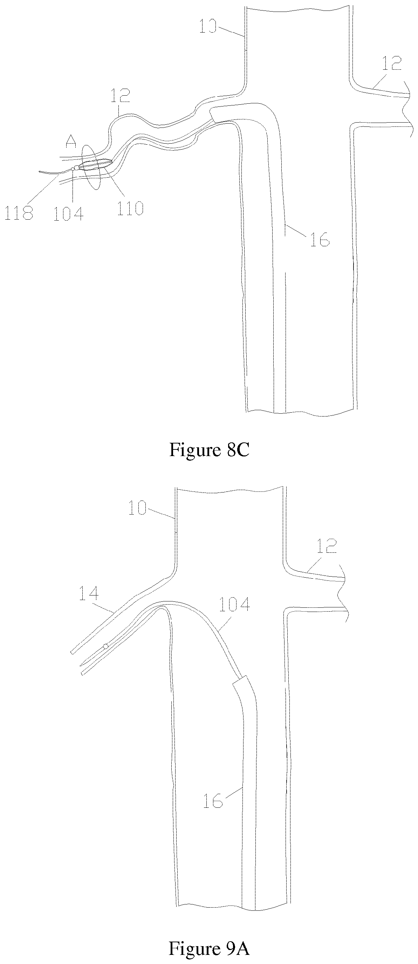

The usage of the support assembly at the distal end of the catheter in bent and complex blood vessels is shown in Figs. 8A, 8B, and 8C. A femoral artery puncture interventional therapy is taken as an example, as shown in Fig. 8A, the doctor firstly makes a small incision at the femoral artery of the thigh of the patient, delivers the guiding wire into the femoral artery via a puncture sheath, pushes the guiding wire into an abdominal aorta 10 with the assistance of angiography, and continuously pushes into the renal artery 12; then pushes the guiding catheter 16 into an introitus to the renal artery 12 along the guiding wire, and withdraws the guiding wire; and then pushes the catheter 100 into the guiding catheter 16, and pushes the support assembly 110 of the catheter 100 out of the guiding catheter 16 to get into the proximal end of the renal artery. Meanwhile, there remains to be a distance from the support assembly 110 to the location of a target site A, but this segment of blood vessel between the introitus of the renal artery 12 and the target site A is bent and complex, and if the surgeon directly pushes the catheter 100 outside the body, it is difficult to push the support assembly 110 to the target site A which is farther away from the introitus of the renal artery 12, in addition, it can easily injure the blood vessels of the renal artery. Meanwhile, the surgeon can establish a transporting path of such a bent and complex blood vessel with the drawing wire 104 of the catheter 100, as shown in Fig. 8B, the surgeon firstly pushes the drawing wire 104 toward the direction of the target site A, because the distal end of the drawing wire 104 is flexible and elongated, the drawing wire 104 will be very smoothly pushed to the target site A, and then the drawing wire 104 is continuously pushed a distance forward, at the same time the surgeon has successfully established the transporting path from the introitus of the renal artery 12 to the target site A. Finally, the surgeon can smoothly push the support assembly 110 to the target site A along the drawing wire 104, as shown in Fig. 8C. Once the support assembly 110 arrives at the target site A, the surgeon operates the drawing wire 104 to move toward the proximal end via a handle 101. The limit unit 118 blocks the distal end of the end 120 so that the drawing wire 104 cannot continuously move relative to the support assembly 110, and the end 120. The drawing wire 104 is continuously operated to move toward the proximal end. As the limit unit 118 blocks the distal end of the end 120, the pull is transferred to the support rods 112 and 113, so that the support rods are elastically bent, thus the support assembly 110 forms an expanded structure, and the electrodes on the support rods will cling to the wall of the blood vessel of the target site A and transports energies to the blood vessel tissue wall, thereby completing the radiofrequency ablation of the renal nerves of the outer wall of the bent and complex renal artery blood vessels.

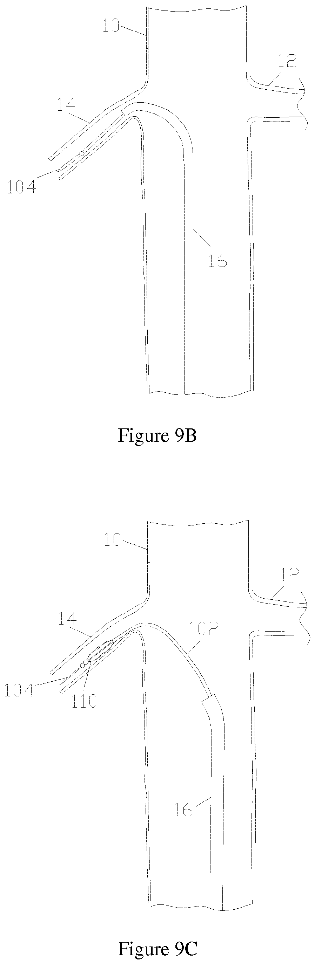

When the renal artery nerves at one side of the patient are ablated completely, the surgeon need to withdraw the catheter 100 into the guiding catheter 16, and withdraw the guiding catheter 16 into abdominal aorta 10 with the assistance of angiography, rotate the guiding catheter 16 into a renal artery 14 at another side of the patient, for some patients with a relatively desirable angle of the renal artery 14 and the abdominal aorta 10, it is easy for the surgeon to be capable of rotating the guiding catheter 16 into the contralateral renal artery 14, however for some patients with an undesirable angle of the renal artery and the abdominal aorta, it is difficult for the surgeon to directly rotate the guiding catheter 16 into the renal artery 14, even if the guiding catheter 16 get into the contralateral renal artery 14, there also may be such a case: as the angle of the renal artery and the abdominal aorta is undesirable, after the catheter 100 is pushed out of the guiding catheter 16 and touches the wall of the blood vessel of the renal artery, the guiding catheter 16 will suffer a counterforce of the wall of the blood vessel of the renal artery to enable it to resile into the abdominal aorta 10, but the catheter 100 can no longer be pushed forward. In this case, the surgeon needs to withdraw both of the guiding catheter 16 and the catheter 100 out of the patient's body, reuses the guiding wire to establish the transporting path of the lateral renal artery, which will increase the operation time of the patient and also cause inconvenience for the surgeon's surgical procedures. However, the drawing wire 104 in the catheter 100 of the present disclosure can replace the above mentioned guiding wire to achieve the purpose of establishing the transporting path, and the particular usage is as shown in Figs. 9A, 9B, and 9C.

In Fig. 9A, after the surgeon has completed the ablation of the renal artery nerves in one side of the patient, the surgeon should withdraw the catheter 100 into the guiding catheter 16, then withdraw the guiding catheter 16 into the abdominal aorta 10, at this time, when the surgeon operates the handle 101 to push the drawing wire 104 out of the guiding catheter 16, the surgeon continues to push the drawing wire 104 with the angiographic technology, as the distal end of the drawing wire 104 is elongated and soft, the drawing wire 104 will get into the contralateral renal artery 14 with a great success, at this time, the surgeon has established the transporting path from the abdominal aorta 10 to the renal artery 14, then the surgeon can push the guiding catheter 16 into the renal artery 14 along the drawing wire 104, as shown in Fig. 9B, the catheter 100 is then pushed out of the guiding catheter 16 to get into the renal artery 14. Otherwise the surgeon can also directly push the support assembly 110 into the renal artery 14, and the guiding catheter 16 remains in the abdominal aorta 10, as shown in Fig. 9C, finally the surgeon completes the radiofrequency ablation of the renal artery nerves through subsequent operations.

The ablation catheter device of the present disclosure, in an aspect, as two support rods of the support assembly at the distal end of the catheter are formed by cutting with tubular products, the cross section thereof is an arc-shaped structure, and after the support assembly is compressed in a sheath tube, the two support rods can form an approximately tubular structure, moreover the wall of the electrodes coating the surface of the support rods is extremely thin, and the size of the support assembly is substantially not increased, while the selected products with the tube diameter being smaller than 1.78 mm (6F refers to the diameter of the guiding tube) are used for making the support rods by cutting, the support assembly composed by the support rods and the electrodes can delivery in the 6F guiding catheter, therefore, the ablation catheter device of the present disclosure not only can perform the radio frequent ablation to the renal artery sympathetic nerves via a femoral artery puncture interventional path, but can also perform the radio frequent ablation to the renal artery sympathetic nerves via a radial artery puncture interventional path, which can bring help for the patients that cannot be performed the femoral artery puncture interventional therapy; in another aspect, the ablation catheter device of the present disclosure has a drawing wire, the distal end of the drawing wire is soft and slender, and which is cooperated with the limit unit to control the radial dilation and the radial compression of the support assembly, without other complex mechanical structures to control the support assembly, thereby not affecting on the structure and size of the support assembly, and ensuring that the support assembly may be applicable to the sheath tube sized as 6F or smaller than 6F, while the drawing wire can also get into the bent and complex blood vessels of the renal artery to directly establish a transporting path, the support assembly at the distal end of the catheter can also get into the bent and complex blood vessels of the renal artery that other ablation catheters cannot get into along the drawing wire to perform the radiofrequency ablation of the renal artery sympathetic nerves, which can bring help for the patients having bent and complex blood vessels of the renal artery.

Over the prior art, the present disclosure has the following significant advantages:
(1) The present disclosure only adopts the drawing wire to cooperate with the limit unit to control the support assembly without other complex mechanical structures, therefore it is easy to operate, and simplifies the manufacturing process; at the same time, the drawing wire is thin, the overall size of the support assembly is not increased after the drawing wire penetrates the space formed by the enclosure of the support assembly, thus the ablation catheter device of the present disclosure can be applicable to the sheath tube sized as 6F or smaller than 6F.
(2) Preferably, the top provided with electrodes on the support rods is arc-shaped, every arc shape cooperates with each other to form an approximately tubular structure, the drawing wire can pass through the inner cavity of the tubular structure, and will not bring influence on the support structure contracted in the sheath tube, further ensuring that the ablation catheter device of the present disclosure can be applicable to the sheath tube sized as 6F and smaller than 6F.
(3) After the catheter is pushed into the target site blood vessel, the drawing wire can be controlled directly as a guiding wire to establish a path within the blood vessels, even though the vascular morphology is complex and bent, the catheter can also be smoothly pushed to the target site.

The above mentioned examples only express several embodiments of the present disclosure, the description thereof is relatively specific and particular, and is not to be construed as limiting the scope of the present disclosure. It will be appreciated that various changes and modifications may be made to the invention as described herein without departing form the spirit and scope thereof. Thus, the protective scope of the present disclosure should be determined by the appended claims.

## Claims

1. An ablation catheter device, comprising:
a hollow catheter main body;
an ablation mechanism including a support assembly capable of being expanded and compressed radially, an end and a plurality of modulation units, wherein the support assembly is provided between the distal end of the main body of the catheter and the end, the plurality of the modulation units are provided on the support assembly, and the end has an axial through hole; and
a control mechanism including a drawing wire and a limit unit fixed on the drawing wire, wherein the drawing wire penetrates the main body of the catheter and the through hole coaxially and movably, the end is provided between the support assembly and the limit unit, and the outer diameter of the limit unit is larger than the pore diameter of the through hole.

2. The ablation catheter device of claim 1, **characterized in that** the support assembly comprises a plurality of elastic support rods isolated from each other, the proximal ends of the support rods are connected to the distal ends of the main body of the catheter, and the distal ends of the support rods are all connected to the end.

3. The ablation catheter device of claim 2, **characterized in that** the support rod comprises a top which is approximately parallel to the main body of the catheter, two inclined parts which incline relative to the main body of the catheter and two bending parts; the modulation units are provided on the top; the two inclined parts are provided on two sides of the top respectively, and are connected to the main body of the catheter and the end respectively; the two bending parts are connected to the top and the two inclined parts respectively; and the rigidity of the bending parts is smaller than that of the top and the inclined parts.

4. The ablation catheter device of claim 3, **characterized in that** the cross section of the top which is perpendicular to the length direction of the main body of the catheter is arc-shaped.

5. The ablation catheter device of claim 4, **characterized in that** tops of the plurality of the support rods cooperate with each other to form an approximately circular ring on the cross section which is perpendicular to the length direction of the main body of the catheter; and the drawing wire movably penetrates the circular ring.

6. The ablation catheter device of claim 3, **characterized in that** on the cross section which is perpendicular to the length direction of the main body of the catheter, the widths of the tops are maximum and the widths of the inclined parts are minimum, and the widths of the bending parts gradually narrow from the tops to the inclined parts.

7. The ablation catheter device of claim 3, **characterized in that** the tops are coated with insulating layers, and the modulation units are provided on the outer surface of the insulating layers.

8. The ablation catheter device of claim 2, **characterized in that** the end comprises an axially provided groove which receives the distal ends of the support rods.

9. The ablation catheter device of claim 1, **characterized in that** the drawing wire is a metal filament, and comprises a flexible distal end, wherein the flexible distal end comprises a winding spring or a polymer sheath.

10. The ablation catheter device of any one of claims 1-9, **characterized by** further comprising a handle provided at the proximal end of the main body of the catheter, wherein the drawing wire is controlled by the handle to move axially.
